(19) Europäisches Patentamt

European Patent Office

Office européen des brevets

(11) **EP 1 658 810 A1**

(12) **EUROPEAN PATENT APPLICATION**

(43) Date of publication:
24.05.2006 Bulletin 2006/21

(51) Int Cl.:
*A61B 5/0484* (2006.01)    *A61B 5/12* (2006.01)

(21) Application number: 05257184.1

(22) Date of filing: 22.11.2005

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 22.11.2004 US 629427 P

(71) Applicant: Vivosonic, Inc.
Toronto,
Ontario M8X 2W4 (CA)

(72) Inventor: Kurtz, Isaac
Toronto, ON M3H 2B3 (CA)

(74) Representative: Evans, Huw David Duncan
Chapman Molony
Cardiff Business Technology Centre
Senghenydd Road
Cardiff
CF24 4AY (GB)

(54) **Method and systems for modulating a steady state stimulus**

(57)    A method and system for modulating a steady state stimulus involving first generating a steady state stimulus signal and then modulating the steady state stimulus signal by at least a portion of a sinusoidal signal where the stimulus is set to zero during another portion of the sinusoidal signal. The steady state stimulus may also be offset by the addition of a constant or may be passed through a bandpass filter to reduce or eliminate frequency components that are outside the desired range of stimulation.

FIG. 3

EP 1 658 810 A1

## Description

### Field of the Invention

This invention relates generally to a method and system for modulating a steady state stimulus and more particularly to a method and system for testing sensory responses of a subject using a modulated stimulus, such as a stimulus for Auditory Steady State Response (ASSR), for auditory testing.

### Background

Living animals generate electrical potentials which, when collected, detected and analysed, can be used for a variety of purposes. For example, synchronous neural activity in a live animal or human brain produces electrical potentials that can be detected at the surface of the scalp with conductive electrodes. These detected potentials can then be used in a wide variety of clinical applications, particularly diagnostic applications.

Stimulation of sensory systems, including but not limited to the auditory and visual systems, with amplitude-modulated (AM) and/or frequency-modulated (FM) tones evoke electrical responses from the brain that correspond to the frequency of modulation. These responses, sustained for the duration of stimulation are known as steady-state responses or steady-state evoked potentials (SSEP). Particularly, Auditory Steady State Responses (ASSR) have been shown to have clinical value in objectively estimating the threshold of hearing at specific frequencies.

An ASSR is an electric sinusoidal signal, which is believed to originate in the brainstem, typically elicited by a modulated sinusoidal stimulus. For example, the stimulus may be a carrier tone of audible frequency range, the amplitude or frequency of which is modulated with a low modulation frequency, typically between 40 and 100 Hz. The ASSR signal evoked then has exactly the same frequency of such modulation. Unfortunately, the ASSR also has a very low amplitude, which causes difficulty in reliably extracting the ASSR signal from noise.

The general principle of ASSR measurement is described as follows. A modulated pure tone is presented to the ear. The carrier frequencies are usually conventional audiometric tones, from 125 to 8000 Hz. The levels of the frequencies are at or higher than 20 dB SPL. The modulation frequencies are typically 40 Hz or within the 70 to 100 Hz range (usually 80 Hz if they are in the 70 to 100Hz range), with a 0.95 modulation index.

At the time of stimulation, a sinusoidal electric signal (the ASSR), which has a frequency equal to the modulation frequency of the stimulus, appears on the surface of the skull. The ASSR can be recorded from the surface of the skull with three electrodes, typically on the vertex, on the temporal bone, and on the lobule. This electric signal, whose magnitude is typically from 40 to 400 nV, is then amplified with a typical gain of approximately 10,000 VN. It is then passed through a band pass filter, with a typical lower frequency cutoff at 10 to 30 Hz and a higher frequency cutoff at 100 to 300 Hz. The filtered signal is then converted into its digital form and processed.

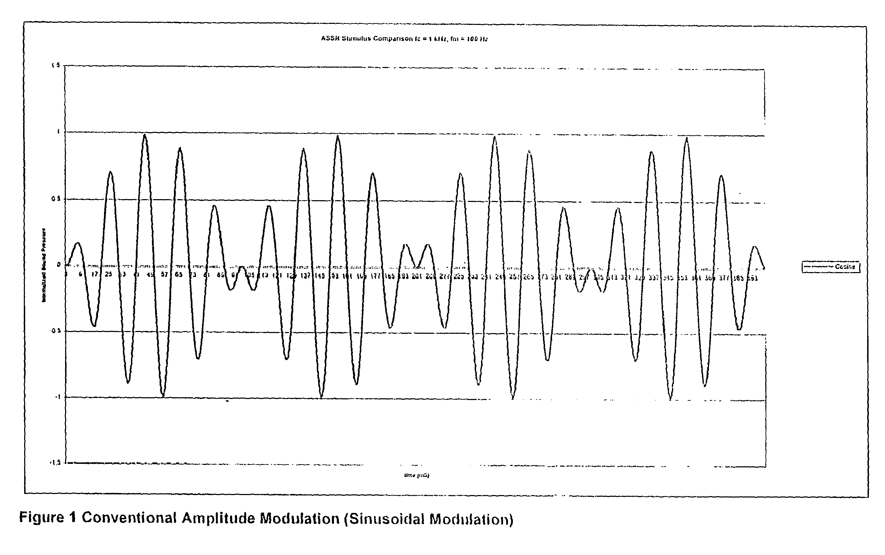

Figure 1 depicts a pure-tone ASSR carrier modulated by a pure sinusoidal modulation in the case where Amplitude Modulation is used. The frequency spectrum of the resulting stimulus waveform contains only the carrier frequency ($f_c$) and the two side-band frequencies ($f_c+f_m$ and $f_c-f_m$). Since the modulation frequency $f_m$ is typically much smaller than $f_c$, only those sensorineural pathways that are sensitive to frequencies very close to the carrier are stimulated. This frequency specificity in hearing threshold estimates is clinically valuable for optimizing intervention in patients with hearing loss. Although ASSR signals have the advantage that they are frequency-specific, as noted above, ASSR signals are difficult to detect in the presence of electromagnetic interference due to the ASSR signal's low amplitude relative to other electrical signals recorded from the scalp, such as electroencephalography (EEG), electrocardiography (ECG), electrooculography (EOG), and electromyography (EMG).

An alternative method for estimating frequency-specific hearing thresholds is to use a transient stimulus that consists of a short tone burst. The auditory brainstem response (ABR) to the tone burst, a response recognizable to trained clinicians, can be detected if the transient sound is above the patient's threshold for hearing. The disadvantage of this method is that a rapid stimulus onset is required to evoke a response that is easier to recognize, but a rapid stimulus onset also results in a broad stimulus spectrum that is not limited to the specific frequency of the tone, i.e. the tone-burst test is not as frequency-specific as the steady-state response is.

In order to achieve better frequency specificity without compromising response level, Gorga et al. (Gorga M. P. et al. (1992), Journal of the American Academy of Audiology, 3, 159-165, "Auditory brainstem responses elicited by 1000 Hz tone bursts in patients with sensorineural hearing loss", which is hereby incorporated herein by reference) explored the use of a variety of tone-burst stimulus onset and offset waveforms. This included the use of a waveform providing a short tone-burst described as a "Blackman Envelope". Because waveforms such as the "Blackman envelope" have broader frequency spectrum in their main frequency lobe but smaller side lobes compared to linearly gated tone bursts, it was presumed by Gorga that these waveforms would produce the effects of a rapid onset stimulus while maintaining more frequency specificity. Purdy et al (Purdy et al. (2002), Ear & Hearing 2002;23;358-368, "ABR Thresholds to tonebursts gated with Balckman and Linear Windows in Adults with High Frequency Sensorineural Hearing Loss", which is hereby incorporated herein by reference) showed, however, that there is no significant difference between linear and Blackman windows in actual threshold determination.

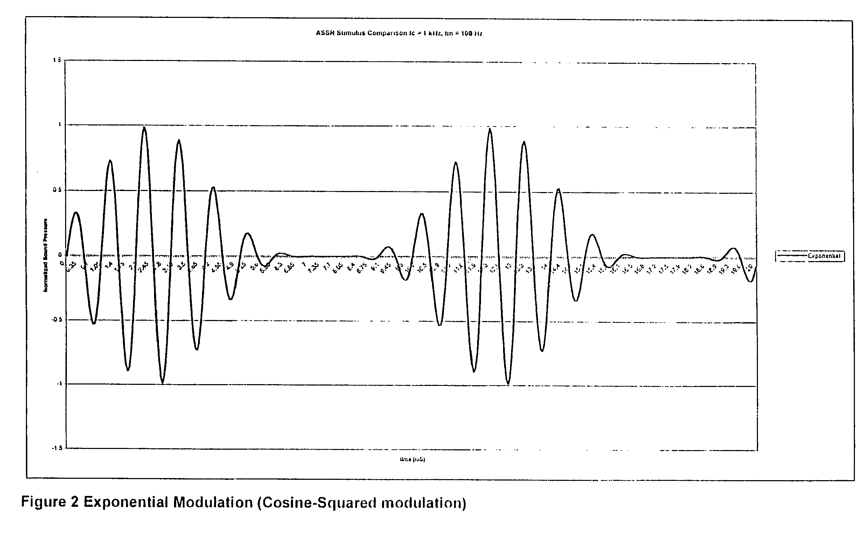

John et al. (John, M. S. et al (2002), Ear and Hearing 2002; 23; 106-117, "Auditory Steady-State Responses to Exponential Modulation Envelopes", which is hereby incorporated herein by reference) explored boosting the Auditory Steady-State Response by using a stimulus consisting of a pure tone modulated by a cosine-squared function. Figure 2 illustrates cosine-squared exponential modulation for the case of amplitude modulation ("AM"). John et al. studied modulation functions using other exponents as well, with similar results. This set of modulation functions is known generally as "exponential modulation". The cosine-squared AM technique has the property that it adds a single harmonic to the fundamental modulation frequency. The resulting waveform has frequency components at fc, fc +/- fm, and fc +/- 2fm, but at no other frequency. John et al. reports that the ASSR signal was enhanced significantly using this technique without compromising frequency specificity. John et al. theorized that the improvement in ASSR performance was possibly due to: the cosine squared function causing the stimulus to be close to zero between peaks allowing for the neurons involved in the response to complete their refractory period before being re-stimulated; and the maximum slope of the modulation signal being greater for the cosine-squared function than that for pure sinusoidal modulation.

While exponential modulation appears to result in good frequency specificity for the stimulus and improves the ASSR response level, this technique requires additional computational complexity in calculating the modulation function. For example, if a lookup table is used to calculate the sine function, the cosine squared function would have to be calculated from this or a separate lookup table would be required. Furthermore, the exponentiation of the cosine function requires more floating-point precision than the calculation of a simple cosine function. The increased computational cost of exponential modulation generally results in additional costs, requires larger equipment, and the like, when used for ASSR related tests. The additional costs and larger equipment may not be as effective in a clinical environment where it can be important to have a steady-state stimulus/ response system, such as an ASSR system, that is, for example, battery-powered, inexpensive, small, lightweight and possibly handheld.

As such, there is a need for an improved stimulus/response system and method and system of modulating a steady state stimulus that overcomes at least some of the disadvantages noted above.

## Summary

The present invention overcomes at least some of the disadvantages of previous approaches by providing a method and system for generating a steady-state stimulus, for example an ASSR stimulus, using a partially rectified cosine as a modulation signal, thus reducing computational complexity, and reducing the size and cost of systems for testing ASSR.

According to one exemplary embodiment, there is provided a method of modulating a steady state stimulus, the method including: generating a steady state stimulus signal; and modulating the steady state stimulus signal by at least a portion of a sinusoidal signal where the stimulus is set to zero during another portion of the sinusoidal signal. In this way, the stimulus provided to a subject is zero for a portion between peaks and it is believed that this allows the neurons involved to complete their refractory period before being re-stimulated.

It will be understood by one of skill in the art that the modulation may be amplitude modulation or frequency modulation.

In a particular case, the portion of the sinusoidal signal may be the positive portion of the sinusoidal signal.

In another particular case, the sinusoidal signal may be offset by the addition of a constant.

In yet another particular case, the sinusoidal signal may be multiplied by a constant greater than 1 to increase to rate of stimulus onset.

In a case where there is an offset and a multiplier, the sinusoidal signal, the offset and the multiplier may be chosen so that the peak, off time and onset rate of the modulation emulate that for cosine squared modulation.

In another particular case, the sensory stimulus may be passed through a bandpass filter to eliminate or reduce frequency components of the stimulus that are outside the desired range of stimulation.

According to another exemplary embodiment, there is provided a system for modulating a steady state stimulus, the system including: a steady state stimulus signal generator; a sine wave signal generator; a rectifier for causing the sine wave signal to be zero for all values of the waveform below a predetermined threshold; and a multiplier for combining the steady state stimulus signal and sine wave signal.

In a particular case, the system further includes an offset generator to offset the sine wave signal by the addition of a constant.

In another particular case, the multiplier mutiplies the sine wave signal by a constant greater than 1 to increase the rate of stimulus onset.

In yet another particular case, the system further includes a bandpass filter to filter frequency components of the stimulus signal that are outside the desired range of stimulation.

According to yet another exemplary embodiment, there is provided a system for testing sensory systems of a subject, the system including: a test signal generator for providing at least one test signal; a modulator for modulating at least one of the at least one test signals by a portion of a sinusoidal signal where the stimulus is set to zero during the other portion of the sinusoidal signal; a transducer for transducing the at least one modulated test signal to create a sensory stimulus, and presenting the stimulus to the subject; a detector for detecting potentials from the subject while substantially simultaneously presenting the stimulus to the subject; and a processor for analyzing the potentials to determine whether the potentials are indicative of the presence of at least one steady-state response to the stimulus.

In a particular case, the portion of the sinusoidal signal is the positive portion of the sinusoidal signal.

In another particular case, the system further includes an offset generator for offsetting the sinusoidal signal by the addition of a constant.

In yet another particular case, the system further includes a multiplier for multiplying the sinusoidal signal by a constant greater than 1 to increase to rate of stimulus onset.

In still yet another particular case, the system further includes a bandpass filter to act on the sensory stimulus to eliminate or reduce frequency components of the stimulus that are outside the desired range of stimulation.

It will be further understood that the modulation described above may be generated by hardware components acting on an analog or digital signal and/or by application of a software program to a digital signal.

### Brief Description of the Drawings

These and other features of the preferred embodiments of the invention will become more apparent in the following detailed description in which reference is made to the appended drawings wherein:

FIG. 1 shows an example of conventional amplitude modulation of a sinusoidal ASSR stimulus;

FIG. 2 shows an example of exponential modulation of a cosine ASSR stimulus;

FIG. 3 shows a side view of an examplary embodiment of stimulus response system;

FIG. 4 shows a top view of the stimulus response system of FIG. 3;

Figure 5 shows an example of the partially rectified cosine wave superimposed on a cosine squared modulated waveform; and

Fig. 6 shows the power spectrum FFT for partially rectified modulation versus that for cosine squared modulation.

### Detailed Description of Embodiments

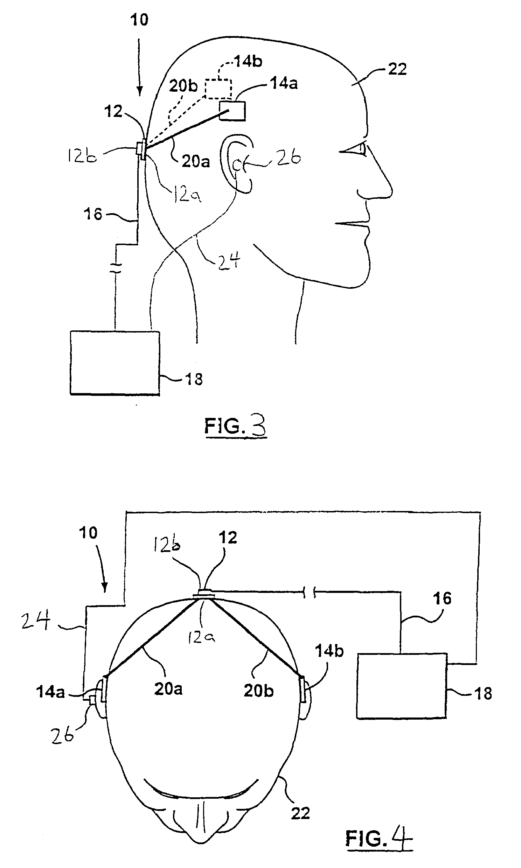

FIG. 3 shows a side view of an examplary stimulus response system 10. Stimulus response system 10 includes a reference electrode module 12. In this exemplary embodiment, reference electrode module 12 includes an electrode 12a and an amplifier component 12b that have been adapted to fit together as a module. Such a reference electrode module is described in greater detail in US Patent Application No. 10/690,630, filed October 23, 2003, by Sokolov et al., which is hereby incorporated herein by reference.

Reference electrode module 12 is electrically coupled to two or more signal electrodes 14. In FIG. 3, two signal electrodes 14 (also referred to herein as simply "electrodes 14") are illustrated - a first electrode 14a is illustrated in the foreground while a second electrode 14b (shown in dotted line) is in the background. Electrodes 14a, 14b are electrically coupled to reference electrode module 12 by lead wires 20a, 20b, respectively.

In this arrangement, reference electrode module 12 also acts as the reference electrical ground for electrodes 14a and 14b. Electrical potentials detected by electrodes 14 will be passed into amplifier component 12b for signal amplification. The use of short lead wires 20 (usually less than 15-20cm in length on an adult human's head and even shorter on an infant's or small animal's head) results in far less noise being inductively or capacitively coupled to the lead wires that carries the signal from electrodes 14 to the amplifier component 12b than conventional electrode-lead and wire-amplifier arrangements. Additionally, since lead wires 20 are preferably taut, motion artifacts that induce noise in the lead wires 20 as they move through static (i.e., time invariant) electromagnetic fields are reduced. The motion artifact noise is reduced compared to known arrangements because lead wires 20a and 20b are likely to move through very similar paths and remain fixed relative to each other through these time-invariant electromagnetic fields. Consequently, there is likely to be only very small differential potentials resulting from these differential motion artifacts that will be detected by amplifier component 12b.

Reference electrode module 12 and electrodes 14 are affixed or mounted to subject 22 through known adhesives or other fixation methods or mechanisms. Additionally, a conductive substance such as electrode gel, for example, may be used to enhance or ensure electrical conduction between the skin of subject 22 and electrodes 12, 14.

Reference electrode module 12 is also electrically coupled to signal processing device 18 by way of connector 16. More particularly, amplifier component 12b is adapted to be electrically connected to lead wires 20a, 20b and connector 16. Connector 16 is preferably a shielded wire allowing amplified electrical potential signals to be transmitted from reference electrode module 12 to signal-processing device 18.

Signal-processing device 18 operates to generate and modulate a steady state stimulus signal and to receive and process signals received from reference electrode module 12 via connector 16. Signal-processing device 18 may be, for example, a conventional signal-processing device that has been adapted to also receive amplified electrical potential signals rather than electrical potentials that have yet to be amplified. Signal-processing device 18 may include, for example, a visual display for displaying the generated and received signals, a signal recorder component for recording the signal received for later review and analysis, and various signal-processing circuits and software for processing any signals generated and received. Such signal processing may include circuitry and/or software for further reducing any noise contained in the received amplified signals.

The signal-processing device 18 is also connected, via transducer connector 24, to a transducer 26. The transducer 26 is for applying the stimulus to the subject 22. In this embodiment, the transducer 26 may be, in this embodiment, an external speaker, a headphone, or an ear inserted speaker, as are known in the art.

Referencing FIG. 4, stimulus response system 10 is illustrated in a top view of subject 22. As noted above, lead wires 20a and 20b electrically connect electrodes 14a, 14b to reference electrode module 12.

Referencing FIGS. 3 and 4, in operation of system 10, an operator affixes reference electrode module 12 and electrodes 14 to a subject in the relevant areas of interest in a manner known to those of ordinary skill in the art. The operator also electrically connects, by way of a lead wire 20, each electrode 14 to reference electrode module 12. In the exemplary embodiment, electrode 14a is connected to reference electrode module 12 by way of lead wire 20a and electrode 14b is connected to reference electrode module 12 by way of lead wire 20b. Lead wires 20 may be connected to electrodes 12, 14 prior or after fixation to the subject. An operator also electrically connects reference electrode module 12 to signal processing device 18 by way of connector 16. The operator then positions the transducer 26 to appropriately deliver the stimulus signal to the subject 22. In this case, by placing an ear insertion speaker.

After the electrodes and transducer (speaker) are arranged appropriately, the stimulus for testing the sensory response of the subject may be applied to the subject.

As described above, the stimulus may be a carrier tone of audible frequency range ("steady state stimulus signal") that is modulated with a low modulation frequency, typically between 40 and 100 Hz. In an exemplary embodiment, a partially rectified cosine wave is applied as a modulation signal rather than the conventional sine function or an exponential cosine function.

The partially rectified cosine wave has the form:

$$ f_m \ = \ \cos(2*pi*f*t)*m/n \ + \ b, $$

for values of t such that - $\cos(2*pi*f*t)*m/n < b$ and $f_m = 0$ for all other values of t.
Here, b is a bias or offset that determines what portion of the modulation signal is nonzero and m/n is a fraction, usually greater than 1, that determines the slope of the onset of modulation.

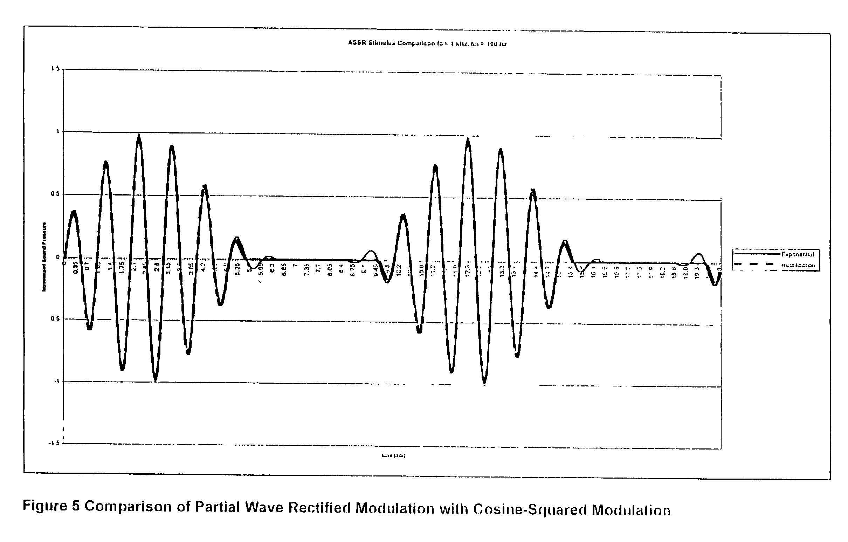

Figure 5 shows an example of the resulting modulated AM waveform (for a value of b=0.5 and m/n=48/33) superimposed on a cosine squared modulated waveform. It is apparent from Fig. 5 that the partially rectified waveform has the advantages of: being zero for a significant portion between peaks allowing for the neurons involved in the response to complete their refractory period before being re-stimulated; and having a maximum slope of the modulation signal that is greater than that for pure sinusoidal modulation.

A further advantage of the present technique is that it requires no additional computational power compared to the calculation of conventional sinusoidal modulation. It will be understood to one of skill in the art that the present technique may be implemented using analog components for the generation of a modulating cosine function, adding the offset "b", rectifying the signal so that the resulting waveform is zero for all negative values of the waveform and multiplying the carrier and modulated signals. Alternatively, the technique may be implemented using software on a digital microcomputer or microcontroller to calculate the function or lookup the function.

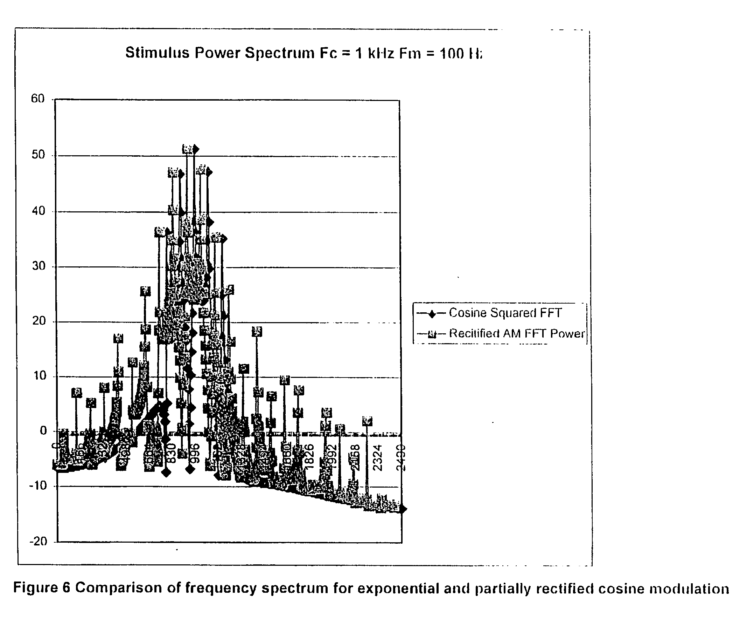

The exemplary embodiment also provides a waveform that is less frequency specific than the cosine squared waveform. FIG. 6 shows the power spectrum FFT for partially rectified modulation versus that for cosine squared modulation. From FIG. 6, it is apparent that, for a 1 kHz carrier, the spectrum of the waveform modulated by a partially rectified cosine contains more power in the range of 500-1500 Hz than the spectrum for the waveform modulated by a cosine squared function. The signal power contained outside this range, however, is small compared to the components close to the center frequency and are not likely to affect threshold detection. In one embodiment of this invention, the stimulus may also be band pass filtered to eliminate or reduce these extra frequency components.

It will be understood that various elements of the embodiments of the invention can be effected by a software program or effected by hardware components or by a combination thereof.

The above embodiments describe a system in which the reference electrode includes an amplifier. It will be understood by one of skill in the art that a more conventional system in which amplification is performed at the signal processing device could also be used.

## Claims

1. A method of modulating a steady state stimulus, said method comprising:

   generating a steady state stimulus signal; and modulating said steady state stimulus signal by at least a portion of a sinusoidal signal where the stimulus is set to zero during another portion of the sinusoidal signal.

**2.** The method of claim 1, wherein said modulation is amplitude modulation.

**3.** The method of either of claim 1 or 2, wherein said portion of the sinusoidal signal is the positive portion of the sinusoidal signal.

**4.** The method of any of claims 1, 2 or 3, wherein said sinusoidal signal is offset by the addition of a constant.

**5.** The method of any of claim 1, 2, 3 or 4, wherein said sinusoidal signal is multiplied by a constant greater than 1 to increase to rate of stimulus onset.

**6.** The method of claim 5, wherein said sinusoidal signal, said offset and said multiplier are set so that the peak, off time and onset rate of the modulation emulate cosine squared modulation.

**7.** The method of any of claims 1,2,3,4,5 and 6, wherein said sensory stimulus is passed through a bandpass filter to eliminate or reduce frequency components of the stimulus that are outside the desired range of stimulation.

**8.** A system for modulating a steady state stimulus, said system comprising:

a steady state stimulus signal generator;
a sine wave signal generator;
a rectifier for causing the sine wave signal to be zero for all values of the waveform below a predetermined threshold; and
a multiplier for combining the steady state stimulus signal and sine wave signal.

**9.** The system of claim 8, further comprising an offset generator to offset said sine wave signal by the addition of a constant.

**10.** The system of either claim 8 or 9, wherein said multiplier mutiplies said sine wave signal by a constant greater than 1 to increase the rate of stimulus onset.

**11.** The system of any of claims 8, 9 or 10, further comprising a bandpass filter to filter frequency components of the stimulus signal that are outside the desired range of stimulation.

**12.** A system for testing sensory systems of a subject, said system comprising:

a test signal generator for providing at least one test signal;
a modulator for modulating at least one of said at least one test signals by a portion of a sinusoidal signal where the stimulus is set to zero during the other portion of the sinusoidal signal;
a transducer for transducing said at least one modulated test signal to create a sensory stimulus, and presenting said stimulus to said subject;
a detector for detecting potentials from said subject while substantially simultaneously presenting said stimulus to said subject; and
a processor for analyzing said potentials to determine whether said potentials are indicative of the presence of at least one steady-state response to said stimulus.

**13.** The system of claim 12, wherein said portion of the sinusoidal signal is the positive portion of said sinusoidal signal.

**14.** The system of either of claim 12 or 13, further comprising an offset generator for offsetting said sinusoidal signal by the addition of a constant.

**15.** The system of any of claims 12, 13, or 14, further comprising a multiplier for multiplying said sinusoidal signal by a constant greater than 1 to increase to rate of stimulus onset.

**16.** The system of claim 15, wherein said sinusoidal signal, said offset and said multiplier are set so that the peak, off time and onset rate of the modulation emulate cosine squared modulation.

**17.** The system of any of claims 12, 13, 14, 15 or 16, further comprising a bandpass filter to act on said sensory stimulus to eliminate or reduce frequency components of the stimulus that are outside the desired range of stimulation.

**18.** The method of any of claims 12 to 17, wherein said modulation is amplitude modulation.

**19.** The method of claim 1, wherein said modulation is effected by a software program.

**20.** The method of claim 1, wherein said modulation is effected by analog components

Figure 1 Conventional Amplitude Modulation (Sinusoidal Modulation)

Figure 2 Exponential Modulation (Cosine-Squared modulation)

FIG.3

FIG.4

Figure 5 Comparison of Partial Wave Rectified Modulation with Cosine-Squared Modulation

Figure 6 Comparison of frequency spectrum for exponential and partially rectified cosine modulation

**European Patent Office**

## EUROPEAN SEARCH REPORT

Application Number

EP 05 25 7184

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JOHN M S ET AL: "MULTIPLE AUDITORY STEADY-STATE RESPONSES TO AM AND FM STIMULI" AUDIOLOGY AND NEURO-OTOLOGY, KARGER, BASEL, CH, vol. 6, no. 1, January 2001 (2001-01), pages 12-27, XP008040668 ISSN: 1420-3030 page 14,15: chapter "Methods" ----- | 1-20 | A61B5/0484 A61B5/12 |
| D,X | JOHN M SASHA ET AL: "Auditory steady-state responses to exponential modulation envelopes." EAR AND HEARING. APR 2002, vol. 23, no. 2, April 2002 (2002-04), pages 106-117, XP009062366 ISSN: 0196-0202 page 107-109: chapter "Amplitude Modulation" ----- | 1-20 | |
| X | US 3 909 705 A (TSCHOPP ET AL) 30 September 1975 (1975-09-30) * column 2, line 29 - column 5, line 2 * * column 10, line 9 - line 42 * * figures 1,2,8 * ----- | 1-5,7-9, 11 | TECHNICAL FIELDS SEARCHED (IPC) A61B |
| X | US 6 524 258 B1 (STUERZEBECHER EKKEHARD ET AL) 25 February 2003 (2003-02-25) * column 1, line 51 - column 2, line 41 * * figure 1 * ----- | 1-3, 12-20 | |
| A | US 2001/049480 A1 (JOHN MICHAEL SASHA ET AL) 6 December 2001 (2001-12-06) * paragraph [0012] - paragraph [0088] * * figure 1 * ----- | 1,8,12 | |

The present search report has been drawn up for all claims

| Place of search | Date of completion of the search | Examiner |
|---|---|---|
| Berlin | 24 February 2006 | Abraham, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 25 7184

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2006

| Patent document cited in search report | | Publication date | Patent family member(s) | | Publication date |
|---|---|---|---|---|---|
| US 3909705 | A | 30-09-1975 | DE | 2237891 A1 | 21-02-1974 |
| | | | JP | 1044847 C | 30-04-1981 |
| | | | JP | 49065279 A | 25-06-1974 |
| | | | JP | 55034898 B | 10-09-1980 |
| US 6524258 | B1 | 25-02-2003 | DE | 19954666 A1 | 13-06-2001 |
| | | | EP | 1099408 A2 | 16-05-2001 |
| US 2001049480 | A1 | 06-12-2001 | US | 2004064066 A1 | 01-04-2004 |

EPO FORM P0459

For more details about this annex : see Official Journal of the European Patent Office, No. 12/82